# EUROPÄISCHE PATENTSCHRIFT

(11) **EP 2 676 681 B1**
(45) Veröffentlichungstag und Bekanntmachung des Hinweises auf die Patenterteilung: **24.12.2014**
(21) Anmeldenummer: 13172468.4
(22) Anmeldetag: 18.06.2013
(51) Int. Cl.: A61L 2/18

(54) **Vorrichtung zur Reinigung und Desinfektion von Reinigungsgut**
Device for cleaning and disinfecting a material to be cleaned
Dispositif de nettoyage et de désinfection d'un produit destiné à être nettoyagé

(30) Priorität: 19.06.2012 DE 102012105328
(43) Veröffentlichungstag der Anmeldung: 25.12.2013
(73) Patentinhaber: MMM MÜNCHENER MEDIZIN MECHANIK GMBH, 82152 Planegg (DE)
(72) Erfinder: Padalko, Oleksandr, 86956 Schongau (DE); Nickel, Kathrin, 82377 Penzberg (DE)
(74) Vertreter: Wittmann, Ernst-Ulrich

(56) Entgegenhaltungen:
- EP-A1- 2 322 858
- WO-A1-2010/128907
- WO-A2-2010/046142
- US-A1- 2013 004 384

## Beschreibung

Die Erfindung betrifft eine Vorrichtung zur Reinigung und Desinfektion von Reinigungsgut mit einem Gehäuse mit einer Vorheizstufe, einer Endheizstufe und einer Reinigungskammer, in das ein Kammergehäuse integriert ist, wobei innerhalb des Kammergehäuses ein Korb zur Aufnahme des Reinigungsguts und mindestens ein Verteilarm zur Verteilung des Reinigungs- und Desinfektionsmittel in dem Kammergehäuse angeordnet ist, wobei die Endheizstufe eine Gebläseeinheit, einen Antrieb für die Gebläseeinheit und ein Heizelement umfasst und wobei die Gebläseeinheit eine Luftturbine aufweist.

Vorrichtung zur Reinigung und Desinfektion von Reinigungsgut dienen zur maschinellen Reinigung, Desinfektion und Trocknung von Medizinprodukten der Klassen I, IIa, IIb sowie Nicht-Medizinprodukten mittels Wasser, Prozesschemikalien und heißer Luft. Solche Vorrichtungen sind beispielsweise auf dem Markt als geschlossene Reinigungs-Desinfektionsgeräte (in der Folge RDG) bekannt. Eine solche Vorrichtung ist in der Regel mit einer Vorrichtung zum Trocknen mit einer Stufe ausgeführt, nämlich einem Ventilator und Schwebstofffilter wie beispielsweise HEPA-Filter (High Efficiency Particulate Airfilter), und mehreren Lufteintrittsöffnungen. Des Weiteren weist die Vorrichtung ein Reinigungssystem auf, das einen Korb zur Aufnahme des Reinigungsguts und als Dreharme ausgestaltete Verteilarme zur Verteilung des Reinigungs- und Desinfektionsmittel in dem Kammergehäuse umfasst.

Bei der Reinigung und Desinfektion von Medizinprodukten ist es erforderlich, die Güter am Ende des Prozesses nach der Desinfektion zu trocknen, um das Risiko einer Rekontamination zu minimieren. Solche Vorgaben sind in den jeweiligen Standards, zum Beispiel in der DIN EN ISO 15883-1, geregelt. Demnach wird zur Trocknung Luft durch die bekannten Vorrichtungen geblasen, welche die Feuchtigkeit der Produkte im Kammergehäuse aufnimmt und nach Außen mit der Abluft abtransportiert. Zur besseren und schnelleren Aufnahme von Feuchtigkeit wird diese Luft mittels des Heizelements aufgeheizt.

Ein wichtiges Kriterium für die Effizienz dieser Ausrüstung ist die Geschwindigkeit mit der die aufgeheizte Luft über das Reinigungsgut geblasen werden kann. Um eine hohe Luftgeschwindigkeit im Inneren der Vorrichtung zu erreichen, muss ein hoher Gesamtvolumenstrom an Luft realisiert werden.

WO2010046142 offenbart ein Reinigungs-/Desinfektionsgerät gemäß dem Oberbegriff des Anspruchs 1.

Im Stand der Technik werden dafür Gebläseeinheiten eingesetzt, die gefilterte Frischluft ansaugen und diese über das Heizelement ins Innere der Vorrichtung einblasen. Die Luft wird hierbei über Luftrohre bzw. -kanäle umgelenkt und derart über spezielle Einlassöffnungen ins Innere des Kammergehäuses eingeblasen, dass eine möglichst gleichmäßige Umströmung des Reinigungsgutes erreicht wird. Durch die umständliche Umlenkung der Luft entstehen Druckverluste im Luft-zufuhr- und Lufteinlasssystem, die durch leistungsstärkere Gebläseeinheiten kompensiert werden müssen.

Der große Luftvolumenstrom bedingt zudem eine entsprechend hohe Heizleistung, um die gesamte Luftmenge aufheizen zu können, und eine entsprechend große Abluftmenge mit feuchter Luft, die von der Klimaanlage abtransportiert werden muss.

Die im Stand der Technik bekannten Nachteile können durch eine kombinierte Bauart des Trocknungssystems aus einem Frischluft- und Umluftsystem deutlich verringert werden. Durch das Erreichen hoher Luftgeschwindigkeiten durch ein Umluftsystem und einer Reduzierung der Menge an Frischluft kann die Frischluftmenge und damit auch die Abluftmenge deutlich reduziert werden. Aus dem Stand der Technik ist bekannt, dass die Vorrichtung zwei Kammern mit eigenen Luftsystemen, einem Frischluft- und einem Umluftsystem, aufweist. Das Frischluftsystem funktioniert wie bereits beschrieben und zusätzlich wird ein zweites System als Umluftsystem um das Kammergehäuse gebaut. Dieses zweite Luftsystem ist von dem Frischluftsystem getrennt als Luftleitungssystem mit Öffnungen für die Luftströmung ausgeführt.

Der vorliegenden Erfindung liegt daher die Aufgabe zugrunde, eine Vorrichtung zur Reinigung und Desinfektion von Reinigungsgut der eingangs genannten Art anzugeben, mittels dessen ein gutes Reinigungsergebnis bei einfacher Konstruktion erzielt werden kann.

Erfindungsgemäß wird die voranstehende Aufgabe durch eine Vorrichtung mit den Merkmalen des Patentanspruchs 1 gelöst. Danach ist das in Rede stehende Vorrichtung zur Reinigung und Desinfektion von Reinigungsgut derart ausgestaltet und weitergebildet, dass die Luftturbine den Luftstrom direkt in das Kammergehäuse einbringt.

In erfindungsgemäßer Weise ist erkannt worden, dass zur Sicherstellung eines guten Reinigungsergebnisses bei einfacher Konstruktion eine Vorrichtung dadurch geschaffen werden muss, dass komplexe Luftführungen vermieden werden können, da diese zum Einen dazu führen, dass leistungsstärkere Gebläseeinheiten oder konstruktiv aufwendige Zwei-Kammer-Systeme verwendet werden müssen, um einen genügend starken Luftstrom zu erzeugen. Zudem sind verunreinigte Stellen, beispielsweise durch Biofilme zu vermeiden, die in den Luftrohren bzw. -kanälen entstehen können, da die Luftrohre bzw. -kanäle nur schwer zugänglich sind und somit nur unzureichend gereinigt werden können. Dies wird durch die erfindungsgemäße, gezielte Modifikation des Verlaufs des Luftstroms in der Art erreicht, dass die Luftturbine den Luftstrom direkt in das Kammergehäuse einbringt, das heißt der Luftstrom nicht über Luftrohre oder -kanäle oder dergleichen von der Luftturbine ins Kammergehäuse einbringbar ist, wobei eine optimale Luftverteilung und/oder vorzugsweise eine direkte Lufterhitzung erfolgen könnte. Hierdurch werden erfindungsgemäß Druckverluste und Ablagerungen in der Luftführung vermieden, so dass kleinere Gebläseeinheiten verwendet werden können, die kostengünstiger sind, und die Vorrichtung gründlicher reinigbar ist. Die Wärmeverluste könnten so minimiert werden. Zudem kann ein komplexes Zwei-Kammersystem vermieden werden, in dem ein automatischer oder manueller Transport des Reinigungsguts von einer Kammer in die andere Kammer zur Trocknung erforderlich ist. Vorzugsweise könnten zwischen den einzelnen Reinigungs-, Spül- und Desinfektionsphasen die Tropfen des Reinigungs- und Desinfektionsmittels am Reinigungsgut abgeblasen werden.

Im Rahmen einer konstruktiv einfachen Ausführungsform könnte die Rotationsachse der Luftturbine im Wesentlichen im rechten Winkel zur Aufnahmeebene des Korbes angeordnet sein, wenn der Korb in dem Kammergehäuse angeordnet ist. Diese Ausgestaltung wäre beispielsweise dann vorteilhaft, wenn die Luftturbine seitlich und/oder im Wesentlichen in der Mitte des Kammergehäuses angeordnet wäre. Die Rotationsachse der Luftturbine könnte jedoch auch in einem spitzen Winkel zur horizontalen Mittelachse des Kammergehäuses oder einer Parallelen hierzu angeordnet sein, zum Beispiel, wenn die Luftturbine seitlich oberhalb zur horizontalen Mittelachse des Kammergehäuses angeordnet ist.

Hinsichtlich einer besonders einfachen Ausführungsform könnte die Luftturbine derart am Kammergehäuse angeordnet sein, dass der im Betrieb durch die Luftturbine erzeugte Luftstrom derart in das Kammergehäuse einströmt, dass eine Luftzirkulation erzeugbar ist, die am Rand der Luftturbine im Wesentlichen horizontal verläuft. Hierdurch ist eine besonders gute Zirkulation der Luft in dem Kammergehäuse und zwischen den einzelnen Etagen des Korbs erreicht, um ein besonders gutes Trocknungsergebnis zu bewirken.

Es könnte - im Rahmen einer wiederum einfachen Ausführungsform - vorteilhaft sein, wenn die Luftturbine in einem zum Kammergehäuse hin offenen Turbinengehäuses angeordnet wäre. Offen heißt in diesem Zusammenhang, dass keine den Durchmesser der Luftturbine wesentlich verkleinernden Elemente zwischen der Luftturbine und dem Inneren des Kammergehäuses liegen, welche den von dem Luftturbine erzeugten Luftstrom erheblich verkleinern, beispielsweise zwischen 5% bis 99,9 %, bevorzugt 20% bis 70%, ganz besonders bevorzugt maximal 30% der freien Fläche der größeren Oberfläche des Turbinengehäuses abdecken. Mit Durchlässen versehende Abdeckungen der Luftturbine, beispielsweise zum Berührschutz, sind daher unter den Begriff offen zu subsumieren.

Hinsichtlich einer besonders guten Reinigungsmöglichkeit könnten der Antrieb der Luftturbine und/oder dessen Lagerung außerhalb des Turbinengehäuse und/oder des Kammergehäuses, angeordnet sein, wobei der Antrieb vorzugsweise mittels einer Gleitringdichtung gegenüber dem Kammergehäuse und/oder dem Turbinengehäuse gedichtet sein könnte. Das Turbinengehäuse könnte somit im Inneren gereinigt werden , ohne dass der Antrieb speziell verkapselt oder sonst wie bei der Reinigung berücksichtigt werden muss.

Im Rahmen einer besonders wartungsarmen Reinigung könnte das Turbinengehäuse kegelstumpfförmig ausgestaltet sein. Die kleinere Bodenfläche des Kegelstumpfs könnte hierbei am dem Ende des Turbinengehäuses angeordnet sein, dass zum Antrieb und die größere Bodenfläche zum Inneren des Kammergehäuses hin orientiert ist, wobei die größere Bodenfläche nicht mit Material verschlossen ist.

Zur Verteilung des Reinigungs- und Desinfektionsmittels in dem Kammergehäuse während des Betriebs könnte der zumindest eine Verteilarm in dem Kammergehäuse angeordnet und/oder rotierbar sein. Es könnten jedoch auch zwei, drei oder mehr Verteilarme vorgesehen sein, wobei ein Verteilarm der im Betrieb oberen Seite des Kammergehäuses angeordnet sein könnte.

Hinsichtlich einer wiederum besonders guten Verteilung des Reinigungs- und Desinfektionsmittels könnten der mindestens eine Verteilarm oder die zwei, drei oder mehr Verteilarme im Wesentlichen horizontal am Kammergehäuse und/oder horizontal an einem Gestell in das der Korb einschiebbar ist angeordnet sein.

Die Verteilung des Reinigungs- und Desinfektionsmittels durch den Verteilarm könnten mittels mindestens einer vertikal angeordneten Sprühdüse, vorzugsweise mehrere im Wesentlichen vertikal angeordnete Sprühdüsen zur Verteilung des Reinigungs- und Desinfektionsmittel, erfolgen. Vertikal heißt in diesem Zusammenhang, dass die Sprühdüsen das Reinigungs- und Desinfektionsmittel in einem sich dreiecksförmig ausweitenden, in Betrieb der Vorrichtung nach unten und/oder oben gerichteten Sprühstrahl verteilen.

Zusätzlich oder alternativ könnten im Endbereich des Verteilarms die Sprühdüsen als Düsenköpfe ausgestaltet sein, die vorzugsweise derart ausgerichtet sind, dass das Reinigungs- und Desinfektionsmittel in das Turbinengehäuse einbringbar ist, so dass die Luftturbine und/oder das Heizelement während des Betriebs gereinigt wird. Die Düsenköpfe könnten hierbei das Reinigungs- und Desinfektionsmittel in einem sich dreiecksförmig ausweitenden, in Betrieb der Vorrichtung seitlich gerichteten Sprühstrahl verteilen. Es könnte jedoch nur ein Düsenkopf vorgesehen sein.

Es gibt nun verschiedene Möglichkeiten, die Lehre der vorliegenden Erfindung in vorteilhafter Weise auszugestalten und weiterzubilden. Dazu ist einerseits auf die im Patentanspruch 1 nachgeordneten Patentansprüche und andererseits auf die nachfolgende Erläuterung eines bevorzugten Ausführungsbeispiels der erfindungsgemäßen Vorrichtung zur Reinigung und Desinfektion von Reinigungsgut anhand der Zeichnung zu verweisen. In Verbindung mit der Erläuterung des bevorzugten Ausführungsbeispiels der erfindungsgemäßen Vorrichtung zur Reinigung und Desinfektion von Reinigungsgut anhand der Zeichnung werden auch im Allgemeinen bevorzugte Ausgestaltungen und Weiterbildungen der Lehre erläutert. In der Zeichnung zeigt die
- Fig. 1: in einer schematischen Ansicht, einen Querschnitt eines Ausführungsbeispiels einer erfindungsgemäßen Vorrichtung zur Reinigung und Desinfektion von Reinigungsgut mit Vorheizstufe, Endheizstufe und Kammer,
- Fig. 2: in einer schematischen Ansicht, im größeren Detail die Endheizstufe und Kammer des Ausführungsbeispiels der Fig. 1.

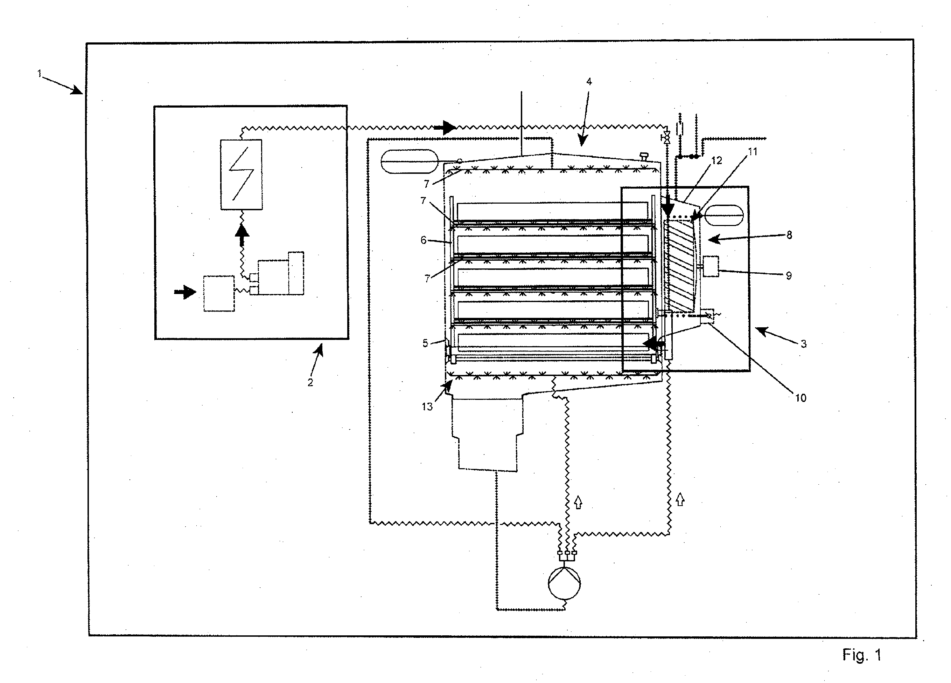

In Fig. 1 ist eine Vorrichtung zur Reinigung und Desinfektion von Reinigungsgut mit einem Gehäuse 1 mit einer Vorheizstufe 2, einer Endheizstufe 3 und einer Reinigungskammer 4 gezeigt, die in ein Kammergehäuse 5 integriert ist. Innerhalb des Kammergehäuses 5 ist ein Gestell angeordnet, in das ein Korb 6 zur Aufnahme des Reinigungsguts eingebracht ist, und fünf Verteilarme 7 zur Verteilung des Reinigungs- und Desinfektionsmittels in dem Kammergehäuse 5. Die Vorheizstufe 2 umfasst einen HEPA-Filter, einen Luftkompressor zum Ansaugen der Luft durch den HEPA-Filter und ein Vorheizelement. Die gereinigte und vorgewärmte Luft wird über eine Verbindung zur Kammer, nämlich zur Endheizstufe 3 geleitet. Die Endheizstufe 3 umfasst eine Gebläseeinheit 8, einen Antrieb 9 für die Gebläseeinheit 8 und ein Heizelement 10, wobei die Gebläseeinheit 8 eine Luftturbine 11 aufweist.

In erfindungsgemäßer Weise ist die Vorrichtung derart ausgestaltet, dass die Luftturbine 11 den Luftstrom direkt in das Kammergehäuse 5 einbringt.

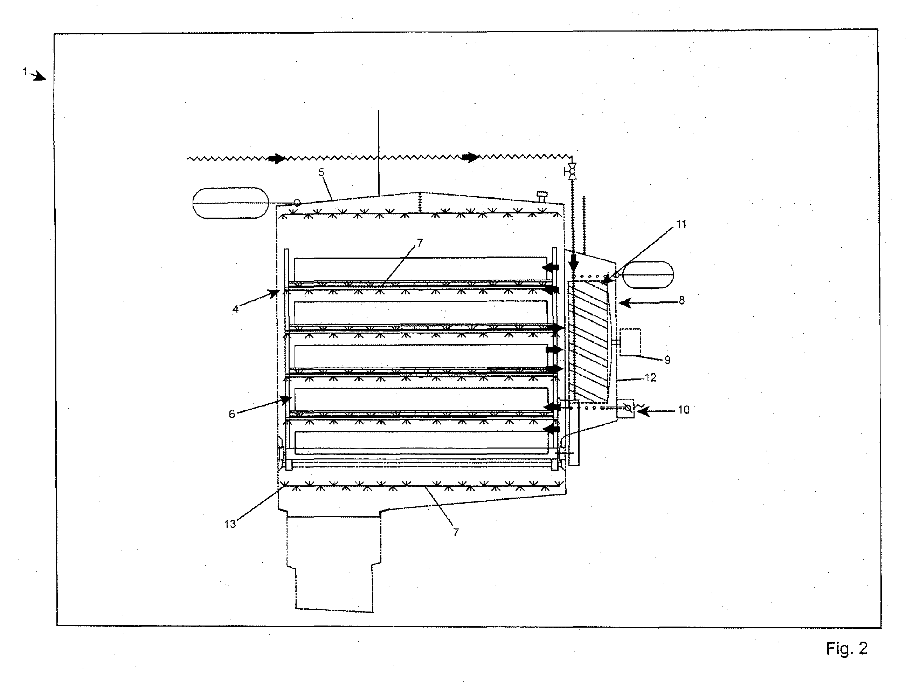

In Fig. 2 ist die Kammer näher gezeigt. In der Endheizstufe 3 ist die Gebläseeinheit 8, der Antrieb 9 für die Gebläseeinheit 8 und das Heizelement 10 gezeigt, wobei die Gebläseeinheit 8 eine Luftturbine 11 umfasst. Die Rotationsachse der Luftturbine 11 ist hierbei im Wesentlichen in einem rechten Winkel zu einer seitlichen Wand des Kammergehäuses 5 angeordnet. Im Betrieb strömt der durch die Luftturbine 11 erzeugte Luftstrom in das Kammergehäuse 5, wobei eine Luftzirkulation - welche stilisiert gemäß der in Fig. 2 gezeigten Pfeile verläuft - erzeugt wird, die am Rand der Luftturbine 11 im Wesentlichen horizontal verläuft. Die Luftturbine 11 ist in einem zum Kammergehäuse 5 hin offenen Turbinengehäuses angeordnet. Auf dem der Kammergehäuse 5 hingewanden Seite des Turbinengehäuses 12 ist eine Abdeckung aus Edelstahl als Berührungsschutz vorgesehen, die max. 30 % der freien Fläche abgedeckt.

Der Antrieb der Luftturbine 11 ist außerhalb des Turbinengehäuse 12 und des Kammergehäuses 5 angeordnet, wobei der Antrieb 9 vorzugsweise mittels einer Gleitringdichtung gegenüber dem Turbinengehäuse 12 gedichtet ist.

Es sind fünf Verteilarme 7 im Wesentlichen horizontal am Kammergehäuse 5 und horizontal an einem Gestell angeordnet, in das der Korb 6 oder verschiedene Korbebenen einschiebbar sind. Die Verteilarme 7 weisen eine Vielzahl von vertikal angeordneten Sprühdüsen 13 zur Verteilung des Reinigungs- und Desinfektionsmittels auf. Im Endbereich des Verteilarms sind die Sprühdüsen 13 als Düsenköpfe ausgestaltet, die derart ausgerichtet sind, dass das Reinigungs- und Desinfektionsmittel bei entsprechender Stellung der Verteilarme 7 in das Turbinengehäuse 12 gespritzt wird. Durch die zylindrische Form des Turbinengehäuses 12 fließt das Reinigungs- und Desinfektionsmittel aus dem Turbinengehäuse 12 ins Kammergehäuse 5, wo es dann in einer Senke gesammelt wird. Es erfolgt somit quasi eine Selbstreinigung und -desinfektion der Luftturbine 11, des Turbinengehäuses 12 und des Heizelements 10 der Endstufe.

Somit ist die Trocknungseinheit erfindungsgemäß als Frischluft- und Umluftsystem ausgestaltet.

## Patentansprüche

1. Vorrichtung zur Reinigung und Desinfektion von Reinigungsgut mit einem Gehäuse (1) mit einer Vorheizstufe (2), einer Endheizstufe (3) und einer Reinigungskammer (4), in das ein Kammergehäuse (5) integriert ist,
wobei innerhalb des Kammergehäuses (5) ein Korb (6) zur Aufnahme des Reinigungsguts und mindestens ein Verteilarm (7) zur Verteilung des Reinigungs- und Desinfektionsmittel in dem Kammergehäuse (5) angeordnet ist, wobei die Endheizstufe (3) eine Gebläseeinheit (8), einen Antrieb (9) für die Gebläseeinheit (8) und ein Heizelement (10) umfasst und
wobei die Gebläseeinheit (8) eine Luftturbine (11) aufweist, **dadurch gekennzeichnet, dass**
die Luftturbine (11) den Luftstrom direkt in das Kammergehäuse (5) einbringt und in einem zum Kammergehäuse (5) hin offenen Turbinengehäuse (12) angeordnet ist, und
im Endbereich des Verteilarms (7) vertikal angeordnete Sprühdüsen (13) als Düsenköpfe ausgestaltet und derart ausgerichtet sind, dass das Reinigungs- und Desinfektionsmittel in das Turbinengehäuse (12), zur Selbstreinigung und Desinfektion des Gebläses (11), des Turbinenhäuses (12) und des Heizelements (10) einbringbar ist.

2. Vorrichtung nach Patentanspruch 1, **dadurch gekennzeichnet, dass** die Rotationsachse der Luftturbine (11) parallel zu den Aufnahmeebenen des Korbes (6) angeordnet ist.

3. Vorrichtung nach Patentanspruch 1 oder 2, **dadurch gekennzeichnet, dass** die Luftturbine (11) derart am Kammergehäuse (5) angeordnet ist, dass der im Betrieb durch die Luftturbine (11) erzeugte Luftstrom derart in das Kammergehäuse (5) einströmt, dass eine Luftzirkulation erzeugbar ist, die am Rand der Luftturbine im Wesentlichen horizontal verläuft.

4. Vorrichtung nach Patentanspruch 1, **dadurch gekennzeichnet, dass** der Antreib (9) der Luftturbine (11) außerhalb des Turbinengehäuse (12) und/oder des Kammergehäuses (5) angeordnet ist, wobei der Antrieb (9) vorzugsweise mittels einer Gleitringdichtung gegenüber dem Kammergehäuse (5) und/oder dem Turbinengehäuse (12) gedichtet ist.

5. Vorrichtung nach Patentanspruch 4, **dadurch gekennzeichnet, dass** das Turbinengehäuse (12) kegelstumpfförmig ausgestaltet ist.

6. Vorrichtung nach einem der Patentansprüche 1 bis 5, **dadurch gekennzeichnet, dass** der Verteilarm (7) in dem Kammergehäuse (5) angeordnet und/oder rotierbar ist.

7. Vorrichtung nach einem der Patentansprüche 1 bis 6, **dadurch gekennzeichnet, dass** mindestens ein Vertellarm (7) horizontal am Kammergehäuse (5) und/oder horizontal am Korb (6) angeordnet ist.

8. Vorrichtung nach einem der Patentansprüche 1 bis 7, **dadurch gekennzeichnet, dass** der Verteilarm (7) mindestens eine vertikal angeordnete Sprühdüse (13), vorzugsweise mehrere vertikal angeordnete Sprühdüsen (13) zur Verteilung des Reinigungs- und Desinfektionsmittel, umfasst.

9. Vorrichtung nach einem der Patentansprüche 1 bis 8, **dadurch gekennzeichnet, dass** die Trocknungseinheit als Frischluft- und Umluftsystem ausgestaltet ist.

## Claims

1. A device for cleaning and disinfecting items to be cleaned, said device comprising a housing (1) with a pre-heating stage (2), a final heating stage (3) and a cleaning chamber (4) into which a chamber housing (5) is integrated,
there being arranged inside the chamber housing (5) a rack (6) for receiving the items to be cleaned, and at least one distribution lever (7) for distributing the cleaning and disinfecting agent in the chamber housing (5), the final heating stage (3) comprising a fan unit (8), a drive (9) for the fan unit (8) and a heating element (10), and
the fan unit (8) including an air turbine (11),
**characterized in that**
the air turbine (11) introduces the air stream directly into the chamber housing (5) and is arranged in a turbine housing (12) which is open towards the chamber housing (5), and
spray nozzles (13) arranged vertically in the end region of the distribution lever (7) are designed as nozzle heads, and are oriented in such a way that the cleaning and disinfecting agent can be introduced into the turbine housing (12) for self-cleaning and disinfection of the fan (11), the turbine housing (12) and the heating element (10).

2. The device of claim 1, **characterized in that** the axis of rotation of the air turbine (11) is arranged parallel to the planes of reception of the rack (6).

3. The device of claim 1 or claim 2, **characterized in that** the air turbine (11) is arranged on the chamber housing (5) in such a way that the airflow generated by the air turbine (11) during operation streams into the chamber housing (5) in such a way that an air circulation can be generated which runs substantially horizontally at the edge of the air turbine.

4. The device of claim 1, **characterized in that** the drive (9) of the air turbine (11) is arranged outside the turbine housing (12) and/or the chamber housing (5), wherein the drive (9) is preferably sealed by a mechanical seal against the chamber housing (5) and/or the turbine housing (12).

5. The device of claim 4, **characterized in that** the turbine housing (12) has a frustoconical design.

6. The device of any of claims 1 to 5, **characterized in that** the distribution lever (7) is arranged and/or rotatable in the chamber housing (5).

7. The device of any of claims 1 to 6, **characterized in that** at least one distribution lever (7) is arranged horizontally on the chamber housing (5) and/or horizontally on the rack (6).

8. The device of any of claims 1 to 7, **characterized in that** the distribution lever (7) comprises at least one vertically arranged spray nozzle (13), preferably multiple vertically arranged spray nozzles (13), for distributing the cleaning and disinfecting agent.

9. The device of any of claims 1 to 8, **characterized in that** the drying unit is designed as a fresh-air and circulating-air system.

## Revendications

1. Dispositif en vue du nettoyage et de la désinfection d'objets à nettoyer, avec un boîtier (1) doté d'un échelon de préchauffage (2), d'un échelon de chauffage final (3) et d'une chambre de nettoyage (4), dans laquelle un boîtier de chambre (5) est intégré,
sachant qu'à l'intérieur du boîtier de chambre (5), un panier (6) est disposé en vue de la réception de l'objet à nettoyé et au moins un bras de distribution (7) en vue de la distribution de l'agent détergent et désinfectant dans le boîtier de chambre (5), sachant que l'échelon de chauffage final (3) comprend une unité de soufflante (8), un entraînement (9) pour l'unité de soufflante (8) et un élément de chauffage (10), et
sachant que l'unité de soufflante (8) présente une turbine d'air (11),
**caractérisé en ce que**
la turbine d'air (11) insère le flux d'air directement dans le boîtier de chambre (5) et est disposée dans un boîtier de turbine (12) ouvert vers le boîtier de chambre (5), et
dans la zone d'extrémité du bras de distribution (7) des buses de pulvérisation disposées à la verticale (13) sont agencées comme têtes de buse et alignées de sorte que l'agent détergent et désinfectant est intégrable dans le boîtier de turbine (12), en vue du nettoyage automatique et de la désinfection de la soufflante (11), du boîtier de turbine (12) et de l'élément de chauffage (10).

2. Dispositif selon la revendication 1, **caractérisé en ce que** l'axe de rotation de la turbine d'air (11) est disposé parallèlement aux plans de réception du panier (6).

3. Dispositif selon les revendications 1 ou 2, **caractérisé en ce que** la turbine d'air (11) est disposée sur le boîtier de chambre (5) de sorte que le flux d'air généré en fonctionnement par la turbine d'air (11) circule à l'intérieur du boîtier de chambre (5) de sorte qu'une circulation d'air est générable, qui se produit principalement à l'horizontale sur le bord de la turbine d'air.

4. Dispositif selon la revendication 1, **caractérisé en ce que** l'entraînement (9) de la turbine d'air (11) est disposé en dehors du boîtier de turbine (12) et/ou du boîtier de chambre (5), l'entraînement (9) étant étanché de préférence au moyen d'une garniture étanche à anneau glissant face au boîtier de chambre et/ou au boîtier de turbine (12).

5. Dispositif selon la revendication 4, **caractérisé en ce que** le boîtier de turbine (12) est agencé en forme tronconique.

6. Dispositif selon une quelconque des revendications 1 à 5, **caractérisé en ce que** le bras de distribution (7) est disposé et/ou rotatif dans le corps de chambre (5).

7. Dispositif selon une quelconque des revendications 1 à 5, **caractérisé en ce qu'**au moins un bras de distribution (7) est disposé à l'horizontale sur le corps de chambre (5) et/ou à l'horizontale sur le panier (6).

8. Dispositif selon une quelconque des revendications 1 à 7, **caractérisé en ce qu'**au moins le bras de distribution (7) comprend au moins une buse de pulvérisation disposée à la verticale (13), de préférence plusieurs buses de pulvérisation disposées à la verticale (13) en vue de la distribution de l'agent détergent et désinfectant.

9. Dispositif selon une quelconque des revendications 1 à 8, **caractérisé en ce que** l'unité de séchage est agencé comme système d'air frais et de circulation d'air.
